(19) Europäisches Patentamt
European Patent Office
Office européen des brevets

(11) EP 0 606 380 B1

(12) **FASCICULE DE BREVET EUROPEEN**

(45) Date de publication et mention de la délivrance du brevet:
**25.11.1998 Bulletin 1998/48**

(21) Numéro de dépôt: **92921565.5**

(22) Date de dépôt: **29.09.1992**

(51) Int Cl.[6]: **C07D 215/04**, C07D 215/233, C07D 405/10, C07D 405/04, C07D 491/048, A61K 31/47 // (C07D491/048, 307:00, 221:00)

(86) Numéro de dépôt international:
**PCT/FR92/00903**

(87) Numéro de publication internationale:
**WO 93/07125 (15.04.1993 Gazette 1993/10)**

(54) **QUINOLEINES 2-SUBSTITUEES POUR LE TRAITEMENT DES LEISHMANIOSES**
SUBSTITUIERTE CHINOLINE FÜR DIE BEHANDLUNG VON LEISMANIOSEN
2-SUBSTITUTED QUINOLINES FOR TREATING LEISHMANIOSES

(84) Etats contractants désignés:
**AT BE CH DE DK ES FR GB GR IE IT LI LU MC NL SE**

(30) Priorité: **03.10.1991 FR 9112174**

(43) Date de publication de la demande:
**20.07.1994 Bulletin 1994/29**

(73) Titulaire: **INSTITUT FRANCAIS DE RECHERCHE SCIENTIFIQUE POUR LE DEVELOPPEMENT EN COOPERATION (ORSTOM)**
**75480 Paris Cédex 10 (FR)**

(72) Inventeurs:
- **FOURNET, Alain**
  **F-40290 Ossages (FR)**
- **ANGELO BARRIOS, Alcira**
  **C.P. 717 La Paz (BO)**
- **MUNOZ, Victoria**
  **C.P. 717 La Paz (BO)**
- **HOCQUEMILLER, Reynald Université Paris Sud**
  **F-92296 Chatenay-Malabry Cédex (FR)**
- **ROBLOT, François**
  **F-75012 Paris (FR)**
- **BRUNETON, Jean**
  **F-49240 Avrille (FR)**
- **RICHOMME, Pascal**
  **F-49000 Angers (FR)**
- **GANTIER, Jean, Charles Faculté de Pharmacie**
  **F-92290 Chatenay-Malabry (FR)**

(74) Mandataire: **Ores, Irène et al**
**CABINET ORES**
**6, Avenue de Messine**
**75008 Paris (FR)**

(56) Documents cités:
**EP-A- 0 326 331** **US-A- 3 636 216**

- **HETEROCYCLES vol. 23, no. 9, 1985, AMSTERDAM pages 2375 - 2385 MINORU ISHIKURA ET AL. 'A simple and regioselective preparation of 2- or 3-substituted Quinoline derivatives via dialkylquinolylboranes' cité dans la demande**
- **PHYTOCHEMISTRY vol. 29, no. 3, 1990, OXFORD pages 813 - 815 PAULO C.VIEIRA ET AL. 'Molluscicidal Quinoline alkaloids from galipea bracteata' cité dans la demande**
- **CANADIAN JOURNAL OF CHEMISTRY vol. 67, 1989, OTTAWA,ONTARIO pages 2116 - 2118 ALAIN FOURNET ET AL. 'Aryl-2 et alkyl-2 quinoléines nouvelles isolées d'une Rutacée bolivienne:Galipea longiflora' cité dans la demande**
- **CHEMICAL ABSTRACTS, vol. 74, no. 16, 19 Avril 1971, Columbus, Ohio, US; abstract no. 79530g, BRIESKORN CARL H ET AL. 'Another alkaloid from angostura bark' page 218 ;colonne 2 ;**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 606 380 B1

Printed by Jouve, 75001 PARIS (FR)

(Cont. page suivante)

- CHEMICAL ABSTRACTS, vol. 75, no. 3, 19 Juillet 1971, Columbus, Ohio, US; abstract no. 20723q, NARASIMHAN,N.S ET AL. 'Synthetic application of lithiation reactions.IV.Novel synthesis of linear Furoquinoline alkaloids and a synthesis of edulitine.' page 490 ;colonne 2 ;
- CHEMICAL ABSTRACTS, vol. 81, no. 1, 8 Juillet 1974, Columbus, Ohio, US; abstract no. 3796x, SHANMUGAM P.ET AL. 'Furoquinolines.VII.Synthesis of furo(2,3-b)quinolines.' page 306 ;colonne 2 ;

## Description

L'Invention est relative à des quinoléines 2-substituées pour le traitement des Leishmanioses.

Les Leishmanioses sont des affections parasitaires dues à des zooflagellés de la famille des Trypano-somidés regroupés dans le genre *Leishmania.*

Les *Leishmania* parasitent le système réticulo-endothélial, en provoquant des atteintes cutanées, cutanéo-muqueuses ou viscérales.

Les traitements des Leishmanioses utilisés jusqu'à présent font essentiellement appel à des sels d'antimoine, en particulier l'antimoniate de N-méthyl glucamine (GLUCANTIME$^{R}$) ; on utilise également des sels de bis(carbonamido-4-phénoxy)1,5 pentane (pentamidine), en particulier pour le traitement des leishmanioses viscérales ; l'amphothéricine B est également utilisée dans les formes graves, résistantes aux autres traitements. Ces traitements présentent l'inconvénient de faire appel à des produits toxiques, peu maniables, car ils doivent être administrés par voie parentérale et présentent des effets secondaires importants, ce qui rend leur utilisation coûteuse, pratiquement impossible en l'absence de structures hospitalières, et donc économiquement inabordable pour la majeure partie des populations des régions touchées par la leishmaniose.

De nombreux travaux ont été effectués dans le but d'obtenir des anti-leishmaniens plus faciles d'emploi que ceux actuellement utilisés. Dans ce but, divers produits déjà connus pour leur activité anti-infectieuse ont fait l'objet d'une évaluation de leur activité biologique sur les leishmanies : il s'agit par exemple d'antifongiques, en particulier du kétoconazole et de ses dérivés, ou d'antibiotiques tels que la rifampicine.

Des antiparasitaires, en particulier des antimalariques ont également été testés : on citera par exemple la primaquine [HANSON et al., Int. J. Parasitol., 7 443-447, (1977) ; BEVERIDGE et al., Trans. R. Soc. Trop. Med. Hyg., 74, 43-51, (1980)] ou des dérivés de celle-ci [SHETTY et al., J. Med. Chem. 21, 995-998, (1978)] et d'autres dérivés de 8-aminoquinoléine tels que les lépidines [KINNAMON et al., Am. J. Trop. Med. Hyg., 27, 751-757, (1977)] ; ces différents composés ont montré des activités antileishmaniennes plus ou moins importantes *in vitro,* et certains d'entre eux possédent également une activité significative *in vivo*, lorsqu'ils sont administrés par voie parentérale ; la quinine et la chloroquine ont également été testées, mais se sont révélées faiblement actives *in vitro* et inactives *in vivo* [MATTOCK et PETERS., Ann. Trop. Med. Parasitol., 69, 359-371, (1975) ; PETERS et al., Ann. Trop. Med. Parasitol., 74, 289-298, (1980) ; PETERS et al., Ann. Trop. Med. Parasitol., 74, 321-335, (1980)]

Récemment, BAUMANN et al. [Antimicrob. Agents Chemoter., 35,1403-1407, (1991)] ont montré l'efficacité *in vivo* par voie parentérale et par voie orale, d'un analogue de polyamine sur *L.donovani ;* cet analogue de polyamine est jusqu'à présent, le seul produit pour lequel une activité significative par voie orale ait été mise en évidence.

Différents traitements à base de plantes sont également utilisés par les populations indigènes dans les régions d'endémie de la Leishmaniose [FOURNET et al. J. Ethnopharmacol., 24, 337 (1988)]. Ces plantes sont en général utilisées en applications locales, sur les lésions.

Les plantes utilisées de la sorte sont plus ou moins bien identifiées sur le plan botanique, et aucune évaluation sérieuse de leur efficacité réelle n'a été effectuée jusqu'à présent.

Les Inventeurs ont entrepris l'étude de différentes plantes préconisées dans la pharmacopée traditionnelle des populations indiennes d'Amérique du Sud pour le traitement de la Leishmaniose. Ils sont de la sorte parvenus à démontrer l'action effective sur les *Leishmania,* d'extraits de *Galipea longiflora* Krause.

*Galipea longiflora* [KRAUSE, Notizbl. K. Bot. Gart. Berlin, 6, 143, (1914)] appartient au genre *Galipea* de la famille des Rutacées. C'est un arbuste de 10 à 15 mètres de haut, présent en Bolivie, et dans les régions limitrophes du Brésil au Nord Est de la Bolivie. Son écorce fraîche réduite en pâte, est utilisée par la médecine traditionnelle locale en cataplasmes pour le traitement topique des lésions cutanées dues à *Leishmania braziliensis,* ainsi qu'en décoction pour le traitement des dysenteries amibiennes.

Certaines espèces du genre *Galipea* ont fait l'objet d'études chimiques ; il s'agit de *Galipea officinalis* Hancock [MESTER, Fitoterapia, 44, 123, (1973)], et récemment de *Galipea bracteata* [VIERA et LUBO, Phytochem. 29, 813, (1990)] et *Galipea longiflora* [FOURNET et al., Can. J. Chem., 67, 2116 (1989)]. Bien que ces études aient permis de mettre en évidence de nombreux constituants de ces plantes, y compris des alcaloïdes quinoléiques, aucun de ces constituants n'a été décrit comme possédant une activité anti-leishmanienne.

Toutefois, les Inventeurs ont poursuivi leurs recherches sur *Galipea longiflora,* dans le but d'identifier des substances responsables de l'activité anti-Leishmanienne.

Leurs travaux ont maintenant abouti à la mise en évidence de différents alcaloïdes quinoléiques dont certains présentent une activité anti-leishmanienne comparable ou supérieure à celle de l'antimoniate de N-méthyl glucamine, pris comme produit de référence.

La présente Invention a pour objet l'utilisation de quinoléines substituées, de formule générale (I)

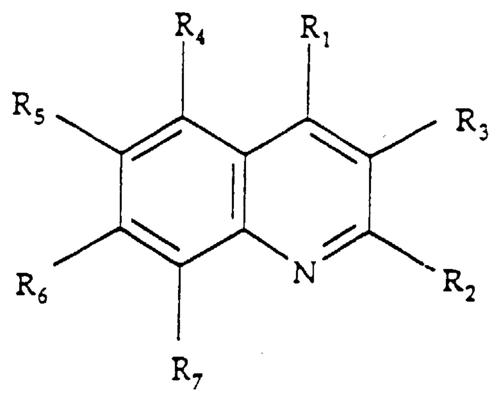

(I)

dans laquelle $R_1$, $R_3$, $R_4$, $R_5$, $R_6$, et $R_7$ représentent chacun indépendamment un atome d'hydrogène, un groupe alkyle, alkényle, époxyalkyle ou mono-ou polyalcool en $C_1$ à $C_7$, linéaire ou ramifié, un groupe amine ou un groupe amide, un groupe OR dans lequel R représente l'hydrogène ou un groupe alkyle ou alkényle en $C_{1-7}$ ou un groupe phényle ; et $R_2$ représente :

- un groupe OR dans lequel R a la même signification que ci-dessus, ou bien
- un groupe alkyle, alkényle, ou époxyalkyle en $C_1$ à $C_7$,
- un groupe phényle, un groupe phénol, méthylènedioxyphényle, diméthoxyphényle, ou bien
- un groupe alkyle, alkényle ou époxyalkyle en $C_{1-7}$ portant au moins l'un des substituants suivants : un groupe alkyle ou alkényle en $C_{1-4}$ ; un groupe phényle, phénol, diméthylphényle, diméthoxyphényle, méthylènedioxyphényle ; ou un groupe OR' dans lequel R' représente l'hydrogène ou un groupe alkyle ou alkényle en $C_{1-4}$ ; ou un groupe NHR" dans lequel R" représente l'hydrogène ou un groupe alkyle ou alkényle en $C_{1-4}$ ; ou un groupe amide, ou bien
- $R_2$ et $R_3$ forment ensemble un cycle furane ; ainsi que les sels et les dérivés desdites quinoléines, pour l'obtention de médicaments antileishmaniens.

Selon un mode de mise en oeuvre préféré de la présente invention, lesdits médicaments sont destinés à l'administration par voie orale.

Parmi les quinoléines anti-leishmaniennes définies par la formule (I) ci-dessus, l'invention a en particulier pour objet :

a) des quinoléines 2-substituées connues en elles-mêmes, mais dont aucune propriété thérapeutique, et en particulier antileishmanienne, n'était connue ou suggérée jusqu'à présent, à savoir :

* une quinoléine de formule (I) dans laquelle $R_3$, $R_4$ et $R_5$ représentent un atome d'hydrogène et $R_1$, $R_6$ et $R_7$ représentent un atome d'hydrogène ou un groupe méthoxy ;
* une quinoléine de formule (I) dans laquelle $R_2$ est un groupe éthyle, éthylène ou époxyéthyle portant au moins l'un des substituants suivants :

  - un groupe alkyle ou alkényle en $C_{1-4}$, ou
  - un groupe OR' dans lequel R' représente l'hydrogène ou un groupe alkyle ou alkényle en $C_{1-4}$,
  - un groupe phényle, phénol, diméthylphényle, diméthoxyphényle, méthylènedioxyphényle ;
  - un groupe amide ou amine ;

* une quinoléine de formule (I) dans laquelle $R_1$, $R_3$, $R_4$, $R_5$, $R_6$, et $R_7$ sont des atomes d'hydrogène ;

  - $R_2$ est un groupe propyle, ou un groupe propényle ;

pour l'utilisation comme médicament ;

b) des quinoléines 2-substituées nouvelles, à savoir :

* une quinoléine de formule (I) dans laquelle :

- $R_3$, $R_4$, $R_5$, $R_6$, et $R_7$ sont des atomes d'hydrogène ;
- $R_1$ est un groupe méthoxy
- $R_2$ est un groupe propyle ;

* une quinoléine de formule (I) dans laquelle :

  - $R_1$, $R_3$, $R_4$, $R_5$, $R_6$, et $R_7$ sont des atomes d'hydrogène ;
  - $R_2$ est un groupe trans-époxypropyle.

Lesdites quinoléines peuvent être extraites d'une plante du genre *Galipea.* Elles peuvent également être obtenues par synthèse chimique, selon tout procédé connu en lui-même, par exemple celui décrit par ISHIKURA et al. [Heterocycles, 23, 2375 (1985)].

Les quinoléines préférées sont celles qui sont 2-substituées par une chaîne à 3 atomes de carbone, saturée ou non, et substituée ou non ; dans ce cadre, l'Invention concerne en particulier la 4-méthoxy-2-n-propyl-quinoléine, dénommée chimanine A, et la 2-(1',2'-trans-époxypropyl)-quinoléine, dénommée chimanine D, qui sont nouvelles, ainsi que la 2-n-propylquinoléine et la 2-propénylquinoléine, qui sont déjà connues [VIEIRA et KUBO ; ISHIKURA et al., publications précitées], mais dont les propriétés antileishmaniennes n'étaient pas connues.

Les quinoléines anti-leishmaniennes conformes à l'Invention sont actives aussi bien sur les leishmanies responsables de la leishmaniose cutanée et cutanéo-muqueuse (par exemple, *Leishmania amazonensis, Leishmania venezuelensis,* etc...), que sur celles responsables de la leishmaniose viscérale *(Leishmania donovani, Leishmania infantum) ;* elles sont utilisables en médecine humaine ou en médecine vétérinaire ; elles sont en outre actives par voie orale.

La présente Invention a en outre pour objet des compositions pharmaceutiques pour le traitement des leishmanioses, caractérisées en ce qu'elles comprennent, en tant que principe actif, au moins une quinoléine de formule (I), choisie parmi les quinoléines nouvelles, ou les quinoléines dont l'utilisation thérapeutique n'avait pas été proposée antérieurement, définies ci-dessus.

Selon un mode de réalisation préféré de la présente Invention, lesdites compositions pharmaceutiques sont formulées pour l'administration par voie orale.

La présente Invention sera mieux comprise à l'aide du complément de description qui va suivre, qui se réfère à des exemples de préparation de quinoléines conformes à l'Invention, et de démonstration de leur activité anti-leishmanienne.

Il va de soi toutefois que ces exemples sont donnés uniquement à titre d'illustration de l'objet de l'Invention, dont ils ne constituent en aucune manière une limitation.

## EXEMPLE I - OBTENTION DE QUINOLEINES ANTI-LEISHMANIENNES A PARTIR DE GALIPEA LONGIFLORA

Les quinoléines ont été obtenues à partir de différents organes (feuilles, écorces des racines et du tronc) de *Galipea longiflora,* séchés et broyés, par extraction à l'éther de pétrole, suivie d'extraction au chloroforme, et purifiées par chromatographie sur colonne de silice H (MERCK) (élution par un mélange hexane-acétate d'éthyle 80/20).

Les formules chimiques respectives des différents alcaloïdes extraits et possédant une activité in vitro sur les leishmanies sont les suivantes :

| | |
|---|---|
| Alcaloïde I : | 2-phénylquinoléine |
| Alcaloïde II : | 2-n-pentylquinoléine |
| Alcaloïde III : | 2-n-propylquinoléine |
| Alcaloïde IV : | 4-méthoxy-2-phénylquinoléine |
| Alcaloïde V : | 2-(3',4'-méthylènedioxyphénéthyl)-quinoléine |
| Alcaloïde VI | : 4-méthoxy-2-n-pentylquinoléine |
| Alcaloïde VII : | 4-méthoxy-2-n-propylquinoléine ou chimanine A |
| Alcaloïde VIII: | 4-méthoxy-2-(3',4'-méthylènedioxyphénéthyl)-quinoléine ou cusparine |
| Alcaloïde IX : | 2-(3',4'-diméthoxyphénéthyl)-quinoléine |
| Alcaloïde X : | 4,7,8-triméthoxy-(2,3-furo)-quinoléine ou skimmianine |
| Alcaloïde XI : | 2-propénylquinoléine ou chimanine B |
| Alcaloïde XII : | 2-(1'2'-trans-époxypropyl)-quinoléine ou chimanine D |
| Alcaloïde XIII: | 4-méthoxy-2-propénylquinoléine ou chimanine C. |

**I - <u>SYNTHESE DE QUINOLEINES 2-SUBSTITUEES</u>**

**EXEMPLE 2 - PREPARATION DE 2-PROPANOL QUINOLEINE ET DE 2-PROPENYL QUINOLEINE, A PARTIR DE 2-METHYL QUINOLEINE**

1) Préparation de 2-propanol quinoléine

Dans un tricol placé sous azote, on met 110 ml de n-butyl lithium 1,6 M dans l'hexane (>0,16 mole), ainsi que 100 ml d'éther anhydre, que l'on refroidit à 0°C. Sous agitation, à l'aide d'une ampoule à brome, on ajoute goutte à goutte 21,65 ml de 2-méthylquinoléine (0,16 mole). On laisse agiter 20 minutes à température ambiante avant de refroidir la solution à -30°C et d'ajouter 13,54 ml d'acétaldéhyde (0,24 mole) par l'ampoule. On rince l'ampoule avec 30 ml d'éther anhydre et on laisse la solution revenir à 0°C.

Au bout de 3 heures, la réaction n'évoluant plus, on hydrolyse par une solution saturée de chlorure d'ammonium. On extrait 3 fois à l'éther. La phase organique est séchée sur sulfate de sodium et évaporée à pression réduite. Le résidu obtenu est chromatographié sur colonne de silice (heptane/acétate d'éthyle : 4/6) pour délivrer 7,5 g de 2-propanol quinoléine et 14,1 g de la quinoléine de départ. (Rdt = 25 %).

2) Préparation de 2-propényl quinoléine

On obtient la 2-propényl quinoléine à partir de la 2-propanol quinoléine par déshydratation dans l'acide acétique en présence d'anhydride acétique.

On dissout dans un ballon surmonté d'un réfrigérant et d'une garde à chlorure de calcium, 468 mg de 2-propanol quinoléine dans un mélange de 0,5 ml (5 mmole) d'anhydride acétique et 0,5 ml (8,3 mmole) d'acide acétique glacial.

On agite la solution au dessus d'un bain de vapeur pendant 45 min. On met un peu d'eau et du carbonate de sodium, puis on neutralise avec une solution de soude 1M. La phase aqueuse est extraite trois fois à l'éther. La phase organique est séchée puis portée à sec par évaporation sous pression réduite. Le résidu obtenu est chromatographié sur silice (hexane/acétate d'éthyle/dichlorométhane : 20/4/76) et on obtient 250 mg de 2-propényl quinoléine (Rdt = 54 %).

**EXEMPLE 3 - PREPARATION DE 2-(2-HYDROXY-2 PHENYLETHYL) QUINOLEINE ET DE 2-STYRYL-QUINOLEINE**

La méthode utilisée est celle de SKIDMORE et TIDD[The Quinoline series, Part I, 1641-1645, (1959)]. On mélange 5,3 ml de 2-méthylquinoléine (0,039 mole) et 3,56 ml de benzaldéhyde (0,035 mole) avec un peu d'acide benzoïque. On agite à température ambiante, puis on laisse le produit cristalliser pendant plusieurs semaines. On filtre la solution sur verre fritté et on rince à l'éthanol froid. Les cristaux restant sur le verre fritté sont recristallisés dans l'éthanol, puis placés dans un dessiccateur chauffant. On obtient ainsi 1,53 g de 2-(2-hydroxy-2 phénylethyl) quinoléine (Rdt = 24%). Les filtrats sont réunis et leur solvant est évaporé, puis les produits sont déposés sur colonne de silice. On obtient alors 3,66 g de 2-méthyl quinoléine et 1,9 g de 2-styryl-quinoléine (Rdt = 18%). On récupère 69% de la 2-méthylquinoléine de départ.

On obtient de plus grandes quantités de 2-styryl-quinoléine en catalysant la déshydratation de la 2-(2-hydroxy-2 phényléthyl) quinoléine par l'acide paratoluènesulfonique, et en éliminant l'eau par entraînement azéotropique.

**EXAMPLE 4 - PREPARATION DE 2-(1',2'-TRANSEPOXYPROPYL) QUINOLEINE**

A une solution de 330 mg de 2-propényl quinoléine (1,96 mmole) dans 12 ml de dioxane et 5 ml d'eau, on ajoute par petites portions 0,42 g de N-bromosuccinimide (2,35 mmole). Après agitation pendant 9 heures à température ambiante, on verse le mélange réactionnel dans l'eau et on l'extrait à l'éther. On sèche sur sulfate de sodium et on évapore l'éther.

Aux deux régioisomères intermédiaires ainsi obtenus, on ajoute 10 ml d'isopropanol, quelques gouttes de phénolphtaléine, et on titre par une solution de soude 1M. La solution est ensuite diluée avec 10 ml d'eau, il se forme alors un précipité qui est extrait 3 fois à l'éther. Le résidu obtenu, après passage de la phase éthérée sur sulfate de sodium et évaporation du solvant, est déposé sur colonne de silice (hexane/acétate d'éthyle : 9/1). On obtient 160 mg de 2-(1',2'-transépoxypropyl) quinoléine (mélange racémique ; Rdt = 44%), et 80 mg (Rdt = 22 %) de 2-dibromopropyl quinoléine).

**EXEMPLE 5 - PREPARATION DE 2-(1',2'-TRANSEPOXYPHENETHYL) QUINOLEINE A PARTIR DE 2-STYRYL QUINOLEINE**

On suit le même mode opératoire qu'à l'exemple 4 en utilisant 1,04 g de 2-styryl quinoléine (4,5 mmole), 0,96 g de N-bromosuccinimide (5,4 mmole) dans 38 ml de dioxane et 16 ml d'eau. On obtient après chromatographie sur silice (hexane/acétate d'éthyle : 8/2), 790 mg (54 %) de 1-phényl-1-bromo-2-(2-quinolyl)éthanol et 70 mg (4%) de 2-[(1,2-dibromo)phénéthyl]quinoléine.

640 mg de 1-phényl-1-bromo-2-(2-quinolyl) éthanol (2,42 mmole), suivent la deuxième partie du traitement. On obtient après purification sur colonne de silice (hexane/acétate d'éthyle : 8/2) 510 mg de 2-(1',2'-transépoxyphénéthyl) quinoléine.

## II. - ACTIVITE BIOLOGIQUE DES ALCALOIDES QUINOLEIQUES CONFORMES A L'INVENTION

**EXEMPLE 6 - ACTIVITE BIOLOGIQUE *IN-VITRO***

Les tests biologiques ont été effectués sur des promastigotes de *Leishmania braziliensis* (souches M 2903 ou M 2904), *Leishmania amazonensis* (souches PH 8 ou H 142) et *Leishmania donovani* (souche M 2682) et sur les épimastigotes de *Trypanosoma cruzi* (souches Tulahuen, C 8CL1, et Tehuentepec).

**a) Culture *in vitro* des *Leishmania* au stade promastigote**

La forme promastigote des différentes souches de *Leishmania ssp.* est maintenue en culture *in vitro* par repiquages successifs (tous les 7 à 15 jours selon les souches) en boites de culture de type "Corning" de 25 ml ou en tubes de culture.

Pour obtenir une meilleure croissance des parasites on ajoute 2 ml du milieu de culture NNN liquide (Novy, Mac Neal, Nicoll) dans une boîte, puis 50 µl de milieu de culture contenant environ 8 x $10^6$ parasites/ml, soit environ 400.000 parasites par boîtes. Cet inoculum est prélevé dans un tube dans lequel la présence de promastigotes a été observée au microscope. Ces parasites sont en grande partie des formes jeunes. On laisse 48 heures à l'étuve à 28°C, et on ajoute de nouveau 2 ml de milieu NNN.

**b) Culture *in vitro* de *Trypanosoma cruzi* au stade épimastigote**

La forme épimastigote des différentes souches de *Trypanosoma cruzi* est maintenue en culture *in vitro* par repiquages successifs chaque semaine environ en boîtes de culture "Corning" de 25 ml ou en tubes de culture.

Les parasites sont cultivés en milieu liquide LIT complet (Yager's Liver Infusion Tryptose) à pH 7,2, supplémenté par 1% de sérum foetal de veau (SVF) inactivé à 56°C pendant 30 minutes et une solution d'antibiotiques contenant 0,25 mg/ml de streptomycine et 250 U/ml de pénicilline.

**c) Essais biologiques *in vitro***

Les tests biologiques sont réalisés dans des microplaques stériles de type "Limbro" ou "Falcon" avec couvercle de 96 puits à fond plat. La capacité de chaque puits est d'environ de 0,35 ml.

Le solvant employé pour dissoudre les extraits de plantes ou les alcaloïdes purifiés à tester est en général le DMSO (diméthylsulfoxide). Avant d'entreprendre les essais avec les extraits de plantes ou les alcaloïdes, la cytotoxicité du DMSO a été évaluée. Il apparaît que ce solvant employé à des concentrations inférieures ou égales à 0,5% n'a aucun effet sur la croissance des parasites.

2 mg d'extrait de plante ou de quinoléine purifiée sont dissous dans 40 µl de DMSO sous agitation (Vortex). A cette préparation est additionnée la quantité nécessaire de milieu NNN (ou bien de milieu LIT dans le cas de *T. cruzi)* pour obtenir la concentration désirée. Les tests destinés à vérifier l'activité des extraits après chaque étape de purification sont effectués à la concentration finale de 100 $\mu g.ml^{-1}$ d'extrait de plante.

Si l'extrait est actif à 100 $\mu g.ml^{-1}$, on recherche la dose minimale qui inhibe complètement les parasites, en préparant des solutions à 50 µg, 25 µg, 5 µg et 1 $\mu g.ml^{-1}$, selon les besoins.

Les parasites sont pris en phase de croissance exponentielle. Le comptage des parasites s'effectue généralement en diluant 10 fois l'inoculum. Un aliquot de la solution contenant les parasites est prélevé puis compté à la cellule de Thoma.

Après ce comptage on ajuste la concentration parasitaire à $10^6$ parasites par ml à l'aide d'une micropipette, puis on dépose 100 µl de parasites dans chaque puits de la microplaque, soit l'équivalent de 100.000 parasites. Ensuite on ajoute la même quantité de milieu de culture (100 µl) contenant l'extrait de plante à la concentration de 200 µg.

$ml^{-1}$. Chaque extrait de plante est testé en triplicate sur chaque souche de parasites et à chaque concentration. Dans les puits témoins contenant les parasites seuls on additionne 100 µl de milieu sans parasite pour arriver à 200 µl. Des puits comprenant des parasites seuls sont également préparés avec le milieu de culture contenant la même quantité de DMSO (40 µl) que pour dissoudre les extraits de plantes, afin de contrôler un éventuel effet du solvant.

Les plaques sont refermées par un couvercle préalablement passé à la flamme afin d'éviter les contaminations, et sont mises à l'étuve à 28°C pendant 48h ou 72h suivant les nécessités de l'expérimentation. Après ce stockage en étuve, les plaques sont examinées au microscope inversé. Chaque puits est observé en le comparant aux puits témoins de culture.

Après 48 ou 72 heures de contact avec les extraits de plantes, on observe au microscope les changements intervenus, et l'on note la concentration qui provoque la lyse totale des parasites.

**d) Résultats**

Les alcaloïdes I, II, V, VI, VIII, IX, X provoquent la lyse totale des cultures de parasites *(Leishmania et Trypanosoma)* à la concentration de 100 µg/ml. Les alcaloides III, IV et XII ont la même action à la dose de 50 µg/ml, et l'alcaloïde XI à la dose de 25 µg/ml.

**EXEMPLE 7 - ACTIVITE *IN VIVO* DES ALCALOIDES CONFORMES A L'INVENTION SUR *L. AMAZONIENSIS* ET *L. VENEZUELENSIS***

**MESURE DE LA TOXICITE DES PRODUITS (DL 50)**

Avant d'effectuer les essais sur les souris, le degré de toxicité des quinoléines a été déterminé.

La méthode choisie est la mesure de la dose léthale 50% (DL50) à dose unique. La DL50 correspond à la dose capable de tuer dans des conditions déterminées, la moitié des animaux mis en expérience dans une même espèce animale. La courbe représentative de la mortalité en fonction du logarithme des doses étant supposée être une droite entre les deux doses qui encadrent la DL50, celle-ci est calculée par interpolation comme suit :

$$\log DL50 = \log.B + \frac{0,5 - N}{M - N} \log \gamma$$

B = dose immédiatement inférieure à la DL50
N = mortalité provoquée par la dose B (pourcentage exprimé en fraction décimale par rapport à 1)
M = mortalité provoquée par la dose immédiatement supérieure à la DL50 (pourcentage exprimé en fraction décimale de 1)
$\gamma$ = raison de la progression.

Les conditions opératoires suivantes ont été choisies :

- Animaux d'expérimentation : les souris Balb/c,
- Nombre d'animaux par concentration de produit : 6 souris de même sexe, de même poids et de même âge,
- Doses d'expérimentation en $mg.kg^{-1}$ : 400, 200, 100, 50, 25, 12,5 (raison de la progression 2),
- voie d'inoculation des produits : intrapéritonéale,
- Calcul de la DL 50 : à temps 0, après 24h, 48h et 72h.

Toutes les quinoléines testées ont une DL 50 supérieure à 400 mg/kg, 72h après l'inoculation des produits. Pour la suite de l'expérimentation, la dose de 100 mg/kg a été employée.

**1. Protocole expérimental**

Chaque groupe expérimental comprend 10 souris Balb/c de même sexe, de même poids et de même âge. Pour chaque expérimentation plusieurs lots expérimentaux sont constitués :

- un lot témoin parasite (Tém.) ne recevant aucun traitement
- un lot témoin parasite recevant pendant la durée du traitement une solution de PBS (Tém. + PBS), le milieu qui contient les parasites
- un lot recevant comme traitement le médicament de référence, l'antimoniate de N-méthyl-glucamine ou GLUCANTIME[R] (commercialisé par SPECIA FRANCE) à raison de 200 $mg.kg^{-1}$

- un lot de souris traitées avec une quinoléine isolée de *Galipea longiflora* ou obtenue par synthèse comme décrit à l'un des exemples 1 à 4, à une concentration de 100 mg/kg.

**2. Souris**

Les animaux d'expérimentation sont des souris Balb/c de 18-24 g, mâles ou femelles âgées de 8 semaines environ. Les souris Balb/c ont été choisies à cause de leur grande sensibilité aux *Leishmania* ssp. et à *Trypanosoma cruzi.*

**3. Maintien des parasites**

Au départ, les formes amastigotes de *L. amazonensis* ont été obtenues par inoculation sous cutanée de promastigotes des souches IFLA/BR/67/PH8 ou MHOM/GF/84/CAY H-142 ou de *L. venezuelensis* (Réf. MHOM/VE/74/PM-H3) au niveau de la face dorsale des pattes arrières de hamsters (Charles Rivers, USA) pesant 120-130 grammes. Les granulomes apparaissent au bout de 8 à 12 semaines suivant la virulence de la souche.

Pour la suite de l'expérimentation, les hamsters sont infectés par les formes amastigotes récupérées dans les granulomes. Par cette méthode les granulomes se développent plus rapidement.

**4. Préparation des parasites et infection**

Pour obtenir des amastigotes, on choisit un hamster présentant un granulome non ulcéré, de taille suffisante. L'animal est tué au chloroforme. La patte arrière infectée par les parasites est nettoyée à l'alcool iodé, puis à l'éthanol à 70°C. A l'aide d'une pince et d'un bistouri stériles, on résèque le granulome. Le granulome est alors haché au-dessus d'un mortier contenant du sable stérile et 5 ml de chlorure de sodium à 0,9%. Le tout est homogénéisé au pilon et la suspension est recueillie à la pipette et centrifugée à 2000 rpm pendant 10 min à 4°C. On obtient trois couches, le surnageant contenant les débris de parasites et de cellules, le fond avec le sable et les gros débris du granulome, et la couche intermédiaire composée de parasites et de globules rouges. Cette couche intermédiaire est prélevée et mise dans un tube, rincée au chlorure de sodium à 0,9% et centrifugée à nouveau pour séparer les globules rouges des parasites qui se présentent sous forme d'une couche blanche.

Les parasites sont repris dans un milieu nutritif MEM (Minimum Essential Medium) avec 10% de SVF (sérum de veau foetal), 2% de L-Glutamine et 1% d'antibiotiques. Ils sont dilués et comptés à la cellule de Thoma. Un granulome permet d'obtenir environ $10^8$ parasites. La concentration est ajustée à $10^6$ parasites par ml. Les parasites ainsi obtenus servent à infecter les souris Balb/c et à réaliser les essais biologiques *in vivo* des produits isolés à partir des plantes. Cette technique d'isolement des parasites est la même pour toutes les souches de *Leishmania* utilisées pour ce travail [*L. amazonensis* (PH8 et H-142) et de *L. venezuelensis* (H-3), Bonfante-Garrido, 1983)].

**5. Infection avec *Leishmania amazonensis et L. venezuelensis***

Les souris infectées par des amastigotes de *Leishmania amazonensis,* des souches de référence IFLA/BR/67/PH8 soit MHOM/GF/84 CAY H 142 ou de *Leishmania venezuelensis* (Référence MHOM/VE/74/PM-H3) provenant de Barquisimeto, Venezuela (hôte : Homme), par voie sous-cutanée au niveau du coussinet ventral de la patte arrière droite, la patte gauche servant de témoin.

La quantité d'amastigotes inoculée est de $10^6$ dans 0,2 ml de PBS pour les souches *Leishmania amazonensis* (pH 8) et *Leishmania venezuelensis* (H-3) et de 3 x $10^6$ amastigotes pour la souche de *Leishmania amazonensis* (H-142) dans le même volume de PBS. Cette concentration de parasites a été préalablement déterminée par des essais à différentes concentrations de parasites.

**6. Traitement avec les principes actifs.**

Les traitements commencent 24 heures après l'inoculation des amastigotes de *Leishmania ssp* et durent 14 jours consécutifs.

L'antimoniate de N-méthyl glucamine est dissous dans de l'eau distillée, et injecté à la dose de 200 $mg.kg^{-1}$ dans 200 µl. La quinoléine à tester est solubilisé dans 40 ml de polysorbate (Tween 80, Prolabo) complété avec du milieu PBS pour obtenir la concentration désirée. Ensuite, 200 µl de la solution obtenue sont inoculés par voie sous-cutanée au niveau dorsal. La concentration du produit dépend de sa toxicité. Peu de problèmes ont été rencontrés lors de l'injection des produits. Il a été observé chez toutes les souris traitées une inflammation locale à l'endroit des piqûres, qui en général disparait à la suspension du traitement.

Des essais d'inoculation locale des médicaments au niveau de la patte infectée ont également été réalisés. Les souris sont infectées de la manière décrite ci-dessus, mais le traitement s'effectue 14 jours après l'infection parasitaire,

et en une seule injection au niveau du coussinet plantaire de la patte arrière infectée par les parasites.

Pour ces essais d'inoculation locale, les concentrations de médicaments administrées sont multipliées par deux. Les médicaments sont préparés de la même manière que lors des traitements par voie générale.

**7. Paramètres étudiés**

Les diamètres des lésions de la patte infectée et de la patte témoin sont mesurés chaque semaine pendant 8 semaines, en commençant les premières mesures 48 heures avant l'inoculation des parasites à l'aide d'un micromètre gradué en 1/10 mm. Le calcul de la différence des deux mesures donne l'épaisseur de la lésion, ou Indice Leishmanien (I.L.).

Les résultats sont exprimés par le calcul de la moyenne arithmétique des Indices Leishmaniens, et le calcul de l'intervalle de confiance de la moyenne observée, au risque $\alpha$ à 5% qui est calculé avec la formule suivante :

$$\text{Intervalle de confiance} = \frac{xs}{\sqrt{n}}$$

x = 2,23 si n(nombre de souris) = 10 au risque $\alpha = 5\%$
x = 2,45 si n = 6 au risque $\alpha = 5\%$
s = la moyenne observée
n = le nombre d'animaux par lot expérimental (10).

Un produit est considéré comme actif lorsque la moyenne des diamètres des lésions des souris traitées avec ce produit est égale ou inférieure à la moyenne des diamètres des lésions des souris traitées avec l'antimoniate de N-méthyl glucamine (GLUCANTIME$^R$).

Les résultats obtenus sur des souris Balb/c infectées par *L. amazonensis* sont représentés sur les figures I à IV.

La figure I(a) illustre l'action de l'alcaloïde III (2-n-propylquinoléine) en traitement général à la dose de 100 mg/kg/jour (■) comparée à celle du GLUCANTIME (♦) à la dose de 200 mg/kg/jour pendant 14 jours consécutifs (début de traitement 24h après infection) ; □ = témoins.

La figure I(b) illustre l'action de l'alcaloïde III en traitement local (une injection unique, 14 jours après l'infection, au niveau de la patte infectée) à la dose de 200 mg/kg (■), comparée à celle du GLUCANTIME$^R$ 400 mg/kg (♦) dans les mêmes conditions de traitement ; □ = témoins.

La figure II illustre l'action de l'alcaloïde VII (4-méthoxy-2-n-propylquinoléine ou chimanine A).

Les figures III(a) et III(b) illustrent l'action de l'alcaloïde XI ( chimanine B) respectivement en traitement général à la dose de 100 mg/kg/jour (■) comparée à celle du GLUCANTIME (♦) à la dose de 200 mg/kg/jour pendant 14 jours consécutifs (début de traitement 24h après infection) ; □ = témoins ; l'action de l'alcaloïde XI (2-n-propénylquinoléine ou chimanine B), en traitement général à la dose de 100 mg/kg/jour (■) comparée à celle du GLUCANTIME (♦) à la dose de 200 mg/kg/jour pendant 14 jours consécutifs (début de traitement 24h après infection) ; □ = témoins ; et en traitement local (une injection unique, 14 jours après l'infection, au niveau de la patte infectée) à la dose de 200 mg/kg (■), comparée à celle du GLUCANTIME$^R$ 400 mg/kg (♦) dans les mêmes conditions de traitement.

La figure IV illustre l'action de l'alcaloïde XII (chimanine D ou 2-(1'2'-trans-époxy-propylquinoléine) en traitement général à la dose de 100 mg/kg/jour (■) comparée à celle du GLUCANTIME$^R$ (♦) à la dose de 200 mg/kg/jour pendant 14 jours consécutifs (début de traitement 24h après infection) ; □ = témoins.

**EXEMPLE 8 - ACTIVITE *IN VIVO* DES ALCALOIDES CONFORMES A L'INVENTION SUR DES SOURIS INFECTEES PAR *LEISHMANIA DONOVANI***

**1 - Protocole expérimental**

Chaque groupe de souris comprend 10 souris BALB/c de même sexe, de même poids et de même âge. Deux protocoles différents ont été effectués et pour chaque expérimentation plusieurs lots expérimentaux ont été constitués :

a) Traitement par voie intrapéritonéale

- un lot témoin non infecté et non traité ;
- un lot de souris témoin infecté et non traité ;
- un lot de souris recevant comme traitement le produit de référence, l'antimoniate de N-méthyl-glucamine ou Glucantine$^R$ à raison de 0,54mM/kg pendant 5 jours consécutifs, soit 200 mg/kg;
- un lot de souris traitées avec une quinoléine à une concentration de 0,54 mM/kg pendant 5 jours consécutifs.

b) Traitement par voie orale ou sous-cutanée

- un lot témoin non infecté et non traité ;
- un lot de souris témoin infecté et non traité ;
- un lot de souris recevant comme traitement le traitement de référence, le Glucantime$^R$ à raison de 0,54 mM/kg pendant 5 jours consécutifs par voie sous cutanée ;
- un lot de souris recevant comme traitement, le Glucantime$^R$ à raison de 0,54 mM/kg pendant 10 jours consécutifs par voie sous cutanée ;
- un lot de souris traitées avec une quinoléine à une concentration de 0,54 mM/kg pendant 5 jours consécutifs par voie sous cutanée ;
- un lot de souris traitées avec une quinoléine à une concentration de 0,54 mM/kg pendant 10 jours consécutifs par voie sous cutanée ;
- un lot de souris traitées avec une quinoléine à une concentration de 0,54 mM/kg pendant 5 jours consécutifs par voie orale ;
- un lot de souris traitées avec une quinoléine à une concentration de 0,54 mM/kg pendant 10 jours consécutifs par voie orale ;

**2 - Souris**

Souris BALB/c de 18 g de sexe femelle provenant de chez IFFA-CREDDO (France).

**3 - Infection par Leishmania donovani**

Les souris infectées par des amastigotes de *Leishmania donovani* (Souche LV/9) par voie intracardiaque. Cette souche provient du laboratoire de Parasitologie de la London School of Hygiene. Les amastigotes sont isolés à partir de foie de hamsters infectés par *L. donovani* (LV9).

La quantité d'amastigotes inoculée est de 2 x $10^7$ dans 0,2 ml de milieu.

**4 - Traitement avec les principes actifs**

Les traitements commencent une semaine après l'infection parasitaire et durent 5 jours ou 10 jours.

Les principes actifs (Glucantime$^R$ et quinoléines) sont dissous dans les mêmes conditions lors des essais *in vivo* décrit à l'exemple 7. En fin d'expérimentation soit 14 jours ou 21 jours après l'infection parasitaire, les souris sont sacrifiées.

**5 - Paramètres étudiés**

Les paramètres suivants sont pris en compte :

- gain de poids des souris (poids initial et poids autopsie) ;
- poids du foie ;
- poids de la rate ;
- comptage au microscope, sur 100 cellules provenant d'imposition du foie sur une lame, du nombre d'amastigotes dans les cellules, et ensuite coloration de cette lame avec le réactif de May-Giemsa ;
- pourcentage de réduction de la parasitémie par rapport aux souris témoins infectées et non traitées.

Les résultats sont exprimés par le calcul de la moyenne arithmétique, de la variance, de l'écart type de chaque paramètre et également du calcul de Stauber qui prend en compte le poids du foie et sont exprimés dans les tableaux I, II et III ci-dessous.

Le Tableau I montre l'activité de quinoléines administrées par voie intrapéritonéale sur des souris BALB/c pendant 5 jours consécutifs, une semaine après infection avec Leishmania donovani (LV/9), en comparaison avec le GLUCANTIME$^R$ administré par voie intrapéritonéale.

Le Tableau II montre l'activité de quinoléines administrées pendant 5 jours ou 10 jours par voie sous cutanée à des souris BALB/c infectées avec *Leishmania donovani* (LV 9), comparée à celle du GLUCANTIME$^R$ administrée par voie sous-cutanée.

Le Tableau III montre l'activité de quelques quinoléines administrées pendant 5 Jours ou 10 jours par voie orale à des souris BALB/c infectées avec *Leishmania donovani* (LV 9), comparée à celle du Glucantime$^R$, administré par voie sous-cutanée.

TABLEAU I

| Nom des produits | Réduction de la parasitémie (%) |
|---|---|
| Glucantime[R] | 98 |
| 2-(1',2'-transépoxypropyl) quinoléine | 57 |
| 2-n-propyl-quinoléine | 62,4 |
| 2-styryle quinoléine | 79,6 |
| 2-(2'-hydroxypropyl) quinoléine | 55,7 |

TABLEAU II

| Nom des produits | Durée traitement en jours | Réduction parasitémie% | Calcul de Stauber |
|---|---|---|---|
| Glucantime | 5<br>10 | 90,5<br>96,9 | 89,9<br>97,4 |
| 2-styryle quinoléine | 5<br>10 | 43,6<br>33,3 | 37,1<br>26,3 |
| 2-(1',2'-transépoxypropyl) quinoléine | 5<br>10 | 69,4<br>87,4 | 69,5<br>86,6 |
| 2-n-propyl-quinoléine | 5<br>10 | 75,9<br>70,6 | 76,3<br>67,8 |

TABLEAU III

| Nom des produits | Durée traitement en jours | Réduction parasitémie % | Calcul de Stauber |
|---|---|---|---|
| Glucantime* | 5<br>10 | 90,5<br>96,9 | 89,9<br>97,4 |
| 2-styryle quinoléine | 5<br>10 | 34,9<br>7,8 | 42,6<br>6,1 |
| 2-(1',2'transépoxypropyl) quinoléine | 5<br>10 | 70,6<br>59,8 | 72,9<br>62,0 |
| 2-n-propyl-quinoléine | 5<br>10 | 87,4<br>70,7 | 87,8<br>99,9 |

* administré par voie sous cutanée

## Revendications

**Revendications pour les Etats contractants suivants : AT, BE, CH, LI, DE, DK, FR, GB, IE, IT, LU, MC, NL, SE**

**1.** Utilisation d'au moins une quinoléine substituée, de formule générale (I)

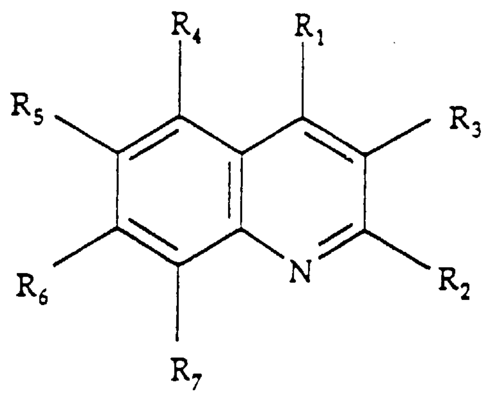

(I)

dans laquelle $R_1$, $R_3$, $R_4$, $R_5$, $R_6$, et $R_7$ représentent chacun séparément un atome d'hydrogène, un groupe alkyle, alkényle, époxyalkyle ou mono-ou polyalcool en $C_1$ à $C_7$, linéaire ou ramifié, un groupe amine ou un groupe amide, un groupe OR dans lequel R représente l'hydrogène ou un groupe alkyle ou alkényle en $C_{1-7}$ ou un groupe phényle ; et $R_2$ représente :

- un groupe OR dans lequel R a la même signification que ci-dessus, ou bien
- un groupe alkyle, alkényle, ou époxyalkyle en $C_1$ à $C_7$,
- un groupe phényle, un groupe phénol, méthylènedioxyphényle, diméthoxyphényle, ou bien
- un groupe alkyle, alkényle ou époxyalkyle en $C_{1-7}$ portant au moins l'un des substituants suivants : un groupe alkyle ou alkényle en $C_{1-4}$ ; un groupe phényle, phénol, diméthylphényle, diméthoxyphényle, méthylènedioxyphényle ; ou un groupe OR' dans lequel R' représente l'hydrogène ou un groupe alkyle ou alkényle en $C_{1-4}$ ; ou un groupe NHR" dans lequel R" représente l'hydrogène ou un groupe alkyle ou alkényle en $C_{1-4}$ ; ou un groupe amide, ou bien
- $R_2$ et $R_3$ forment ensemble un cycle furane ; ou d'un sel ou dérivé de ladite quinoléine pour l'obtention d'un médicament anti-leshmanien.

**2.** Quinoléine substituée de formule (I) telle que définie dans la Revendication 1, dans laquelle $R_3$, $R_4$ et $R_5$ représentent un atome d'hydrogène et $R_1$, $R_6$ et $R_7$ représentent un atome d'hydrogène ou un groupe méthoxy, pour l'utilisation comme médicament.

**3.** Quinoléine substituée de formule (I) telle que définie dans la Revendication 1, dans laquelle $R_2$ est un groupe éthyle, éthylène ou époxyéthyle, portant au moins l'un des substituants suivants :

- un groupe alkyle ou alkényle en $C_{1-4}$, ou
- un groupe OR' dans lequel R' représente l'hydrogène ou un groupe alkyle ou alkényle en $C_{1-4}$,
- un groupe phényle, phénol, diméthylphényle, diméthoxyphényle, méthylènedioxyphényle
- un groupe amide ou amine,

pour l'utilisation comme médicament.

**4.** Quinoléine substituée de formule (I) telle que définie dans la Revendication 1, caractérisée en ce que :

- $R_3$, $R_4$, $R_5$, $R_6$, et $R_7$ sont des atomes d'hydrogène ;
- $R_1$ est un groupe méthoxy
- $R_2$ est un groupe propyle.

**5.** Quinoléine substituée de formule (I) telle que définie dans la Revendication 1, caractérisée en ce que :

- $R_1$, $R_3$, $R_4$, $R_5$, $R_6$, et $R_7$ sont des atomes d'hydrogène ;
- $R_2$ est un groupe trans-époxypropyle.

**6.** Quinoléine substituée de formule (I) telle que définie dans la Revendication 1, dans laquelle :

- $R_1$, $R_3$, $R_4$, $R_5$, $R_6$, et $R_7$ sont des atomes d'hydrogène ;
- $R_2$ est un groupe propyle pour l'utilisation comme médicament.

**7.** Quinoléine substituée de formule (I) telle que définie dans la Revendication 1, dans laquelle :

- $R_1$, $R_3$, $R_4$, $R_5$, $R_6$, et $R_7$ sont des atomes d'hydrogène ;
- $R_2$ est un groupe propényle pour l'utilisation comme médicament.

**8.** Composition pharmaceutique pour le traitement des Leishmanioses, caractérisée en ce qu'elle comprend, en tant que principe actif, au moins une quinoléine telle que définie dans l'une quelconque des Revendications 2 à 7.

**9.** Composition pharmaceutique pour le traitement des Leishmanioses, selon la revendication 8, caractérisée en ce qu'elle est destinée à l'administration orale.

**10.** Utilisation selon la revendication 1, caractérisée en ce que ledit médicament anti-leishmanien est destiné à l'administration orale.

**Revendications pour les Etats contractants suivants : ES, GR**

**1.** Procédé de préparation d'un médicament anti-leishmanien, caractérisé en ce que l'on utilise en tant que constituant actif dudit médicament, une quinoléine substituée, de formule générale (I)

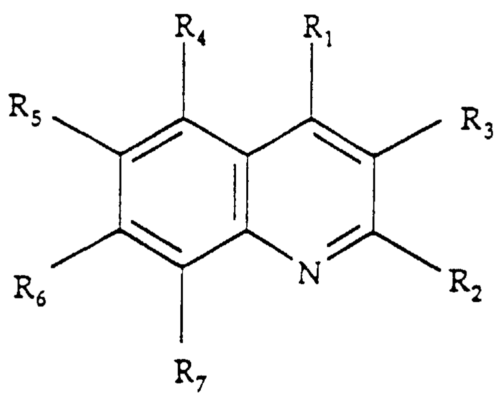

(I)

dans laquelle $R_1$, $R_3$, $R_4$, $R_5$, $R_6$, et $R_7$ représentent chacun séparément un atome d'hydrogène, un groupe alkyle, alkényle, époxyalkyle ou mono-ou polyalcool en $C_1$ à $C_7$, linéaire ou ramifié, un groupe amine ou un groupe amide, un groupe OR dans lequel R représente l'hydrogène ou un groupe alkyle ou alkényle en $C_{1-7}$ ou un groupe phényle ; et $R_2$ représente :

- un groupe OR dans lequel R a la même signification que ci-dessus, ou bien
- un groupe alkyle, alkényle, ou époxyalkyle en $C_1$ à $C_7$,
- un groupe phényle, un groupe phénol, méthylènedioxyphényle, diméthoxyphényle, ou bien
- un groupe alkyle, alkényle ou époxyalkyle en $C_{1-7}$ portant au moins l'un des substituants suivants : un groupe alkyle ou alkényle en $C_{1-4}$ ; un groupe phényle, phénol, diméthylphényle, diméthoxyphényle, méthylènedioxyphényle ; ou un groupe OR' dans lequel R' représente l'hydrogène ou un groupe alkyle ou alkényle en $C_{1-4}$ ; ou un groupe NHR" dans lequel R" représente l'hydrogène ou un groupe alkyle ou alkényle en $C_{1-4}$ ; ou un groupe amide, ou bien
- $R_2$ et $R_3$ forment ensemble un cycle furane ; ou un sel ou dérivé de ladite quinoléine.

**2.** Procédé de préparation d'un médicament, caractérisé en ce que l'on utilise en tant que constituant actif dudit médicament une quinoléine substituée de formule (I) telle que définie dans la Revendication 1, dans laquelle $R_3$, $R_4$ et $R_5$ représentent un atome d'hydrogène et $R_1$, $R_6$ et $R_7$ représentent un atome d'hydrogène ou un groupe

méthoxy.

**3.** Procédé de préparation d'un médicament, caractérisé en ce que l'on utilise en tant que constituant actif dudit médicament, une quinoléine substituée de formule (I) telle que définie dans la Revendication 1, dans laquelle $R_2$ est un groupe éthyle, éthylène ou époxyéthyle, portant au moins l'un des substituants suivants :

- un groupe alkyle ou alkényle en $C_{1-4}$, ou
- un groupe OR' dans lequel R' représente l'hydrogène ou un groupe alkyle ou alkényle en $C_{1-4}$,
- un groupe phényle, phénol, diméthylphényle, diméthoxyphényle, méthylènedioxyphényle
- un groupe amide ou amine.

**4.** Procédé de préparation d'une quinoléine substituée, caractérisé en ce que l'on prépare une quinoléine de formule (I) telle que définie dans la Revendication 1, dans laquelle :

- $R_3$, $R_4$, $R_5$, $R_6$, et $R_7$ sont des atomes d'hydrogène ;
- $R_1$ est un groupe méthoxy
- $R_2$ est un groupe propyle.

**5.** Procédé de préparation d'une quinoléine substituée, caractérisé en ce que l'on prépare une quinoléine de formule (I) telle que définie dans la Revendication 1, dans laquelle :

- $R_1$, $R_3$, $R_4$, $R_5$, $R_6$, et $R_7$ sont des atomes d'hydrogène ;
- $R_2$ est un groupe trans-époxypropyle.

**6.** Procédé de préparation d'un médicament, caractérisé en ce que l'on utilise en tant que constituant actif dudit médicament, une quinoléine substituée de formule (I) telle que définie dans la Revendication 1, dans laquelle :

- $R_1$, $R_3$, $R_4$, $R_5$, $R_6$, et $R_7$ sont des atomes d'hydrogène ;
- $R_2$ est un groupe propyle.

**7.** Procédé de préparation d'un médicament, caractérisé en ce que l'on utilise en tant que constituant actif dudit médicament, une quinoléine substituée de formule (I) telle que définie dans la Revendication 1, dans laquelle :

- $R_1$, $R_3$, $R_4$, $R_5$, $R_6$, et $R_7$ sont des atomes d'hydrogène ;
- $R_2$ est un groupe propényle.

**8.** Procédé selon la revendication 1, caractérisé en ce que l'on utilise en tant que constituant actif dudit médicament, au moins une quinoléine telle que définie dans l'une quelconque des Revendications 2 à 7.

**9.** Procédé selon la revendication 1, caractérisé en ce que ledit médicament anti-leishmanien est destiné à l'administration orale.

## Patentansprüche

## Patentansprüche für folgende Vertragsstaaten : AT, BE, CH, LI, DE, DK, FR, GB, IE, IT, LU, MC, NL, SE

**1.** Verwendung mindestens eines substituierten Chinoleins der allgemeinen Formel (I)

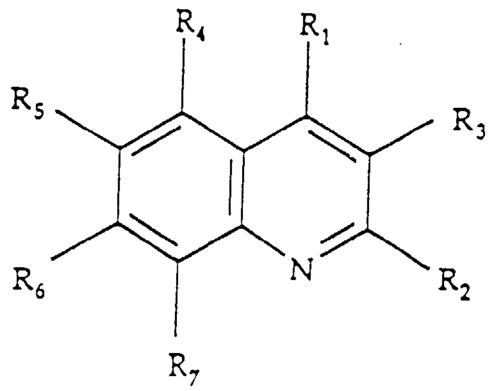

(I)

worin $R_1$, $R_3$, $R_4$, $R_5$, $R_6$ und $R_7$ jeweils getrennt ein Wasserstoffatom, einen linearen oder verzweigten Alkyl-, Alkenyl-, Epoxyalkylrest oder linearen oder verzweigten $C_1$-$C_7$-Mono- oder Polyalkohol, einen Aminrest oder Amidrest, einen OR-Rest, worin R Wasserstoff oder einen $C_1$-$C_7$-Alkyl- oder Alkenylrest oder einen Phenylrest bedeutet, und $R_2$

- einen OR-Rest bedeutet, worin R wie vorstehend definiert ist, oder
- einen $C_1$-$C_7$-Alkyl-, Alkenyl- oder Epoxyalkylrest,
- einen Phenylrest, einen Phenol-, Methylendioxyphenyl-, Dimethoxyphenylrest oder
- einen $C_1$-$C_7$-Alkyl-, Alkenyl- oder Epoxyalkylrest, der mindestens einen der folgenden Substituenten enthält: $C_1$-$C_4$-Alkyl- oder Alkenylrest, Phenyl-, Phenol-, Dimethylphenyl-, Dimethoxyphenyl-, Methylendioxyphenylrest, OR'-Rest, worin R' Wasserstoff oder einen $C_1$-$C_4$-Alkyl- oder Alkenylrest bedeutet, NHR"-Rest, worin R" Wasserstoff oder einen $C_1$-$C_4$-Alkyl- oder Alkenylrest bedeutet oder einen Amidrest bedeutet, bedeutet oder
- $R_2$ und $R_3$ zusammen einen Furanring bilden,

oder ein Salz oder Derivat des Chinoleins zur Herstellung eines Medikaments gegen die Leishmaniose.

**2.** Substituiertes Chinolein der Formel (I) nach Anspruch 1, worin $R_3$, $R_4$ und $R_5$ ein Wasserstoffatom bedeuten und $R_1$, $R_6$ und $R_7$ ein Wasserstoffatom oder eine Methoxygruppe bedeuten, zur Verwendung als Medikament.

**3.** Substituiertes Chinolein der Formel (I) nach Anspruch 1, worin $R_2$ eine Ethyl-, Ethylen- oder Epoxyethylgruppe, die mindestens einen der folgenden Substituenten

- einen $C_1$-$C_4$-Alkyl- oder Alkenylrest oder
- einen OR'-Rest, worin R' Wasserstoff oder einen $C_1$-$C_4$-Alkyl- oder Alkenylrest bedeutet,
- einen Phenyl-, Phenol-, Dimethylphenyl-, Dimethoxyphenyl-, Methylendioxyphenylrest,
- einen Amid- oder Aminrest enthält, bedeutet, zur Verwendung als Medikament.

**4.** Substituiertes Chinolein der Formel (I) nach Anspruch 1, dadurch gekennzeichnet, daß

- $R_3$, $R_4$, $R_5$, $R_6$ und $R_7$ Wasserstoffatome sind,
- $R_1$ eine Methoxygruppe ist,
- $R_2$ eine Propylgruppe ist.

**5.** Substituiertes Chinolein der Formel (I) nach Anspruch 1, dadurch gekennzeichnet, daß

- $R_1$ , $R_3$ , $R_4$ , $R_5$, $R_6$ und $R_7$ Wasserstoffatome sind,
- $R_2$ eine Trans-Epoxypropylgruppe ist.

**6.** Substituiertes Chinolein der Formel (I) nach Anspruch 1, worin

- $R_1$ , $R_3$ , $R_4$ , $R_5$ , $R_6$ und $R_7$ Wasserstoffatome sind,
- $R_2$ eine Propylgruppe ist

zur Verwendung als Medikament.

**7.** Substituiertes Chinolein der Formel (I) nach Anspruch 1, worin

- $R_1$ , $R_3$ , $R_4$ , $R_5$ , $R_6$ und $R_7$ Wasserstoffatome sind,
- $R_2$ eine Propenylgruppe ist

zur Verwendung als Medikament.

**8.** Pharmazeutisches Präparat zur Behandlung von Leishmaniosen, dadurch gekennzeichnet, daß es als Wirkstoff mindestens ein Chinolein nach einem der Ansprüche 2 bis 7 umfaßt.

**9.** Pharmazeutisches Präparat zur Behandlung von Leishmaniosen nach Anspruch 8, dadurch gekennzeichnet, daß es für die orale Verabreichung bestimmt ist.

**10.** Verwendung nach Anspruch 1, dadurch gekennzeichnet, daß das Medikament gegen die Leishmaniose für die orale Verabreichung bestimmt ist.

**Patentansprüche für folgende Vertragsstaaten : ES, GR**

**1.** Verfahren zur Herstellung eines Medikaments gegen die Leishmaniose, dadurch gekennzeichnet, daß man als Wirkstoff des Medikaments ein substituiertes Chinolein der allgemeinen Formel (I)

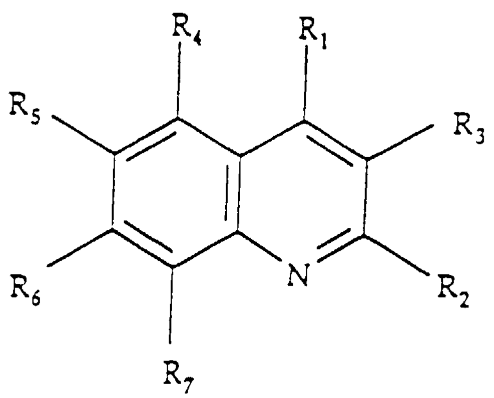

(I)

worin $R_1$ , $R_3$ , $R_4$ , $R_5$ , $R_6$ und $R_7$ jeweils getrennt ein Wasserstoffatom, einen linearen oder verzweigten Alkyl-, Alkenyl-, Epoxyalkylrest oder linearen oder verzweigten $C_1$-$C_7$-Mono- oder Polyalkohol, einen Aminrest oder Amidrest, einen OR-Rest, worin R Wasserstoff oder einen $C_1$-$C_7$-Alkyl- oder Alkenylrest oder einen Phenylrest bedeutet, und $R_2$

- einen OR-Rest bedeutet, worin R wie vorstehend definiert ist, oder

- einen $C_1$-$C_7$-Alkyl-, Alkenyl- oder Epoxyalkylrest,
- einen Phenylrest, einen Phenol-, Methylendioxyphenyl-, Dimethoxyphenylrest oder
- einen $C_1$-$C_7$-Alkyl-, Alkenyl- oder Epoxyalkylrest, der mindestens einen der folgenden Substituenten enthält: $C_1$-$C_4$-Alkyl- oder Alkenylrest, Phenyl-, Phenol-, Dimethylphenyl-, Dimethoxyphenyl-, Methylendioxyphenylrest, OR'-Rest, worin R' Wasserstoff oder einen $C_1$-$C_4$-Alkyl- oder Alkenylrest bedeutet, NHR"-Rest, worin R" Wasserstoff oder einen $C_1$-$C_4$-Alkyl- oder Alkenylrest bedeutet oder einen Amidrest bedeutet, bedeutet, oder
- $R_2$ und $R_3$ zusammen einen Furanring bilden,

oder ein Salz oder Derivat des Chinoleins verwendet.

**2.** Verfahren zur Herstellung eines Medikaments, dadurch gekennzeichnet, daß man als Wirkstoff des Medikaments ein substituiertes Chinolein der Formel (I) nach Anspruch 1 verwendet, worin $R_3$ , $R_4$ und $R_5$ ein Wasserstoffatom bedeuten und $R_1$ , $R_6$ und $R_7$ ein Wasserstoffatom oder eine Methoxygruppe bedeuten.

**3.** Verfahren zur Herstellung eines Medikaments, dadurch gekennzeichnet, daß man als Wirkstoff des Medikaments ein substituiertes Chinolein der Formel (I) nach Anspruch 1, worin $R_2$ eine Ethyl-, Ethylen- oder Epoxyethylgruppe, die mindestens einen der folgenden Substituenten

- einen $C_1$-$C_4$-Alkyl- oder Alkenylrest oder
- einen OR'-Rest, worin R' Wasserstoff oder einen $C_1$-$C_4$-Alkyl- oder Alkenylrest bedeutet,
- einen Phenyl-, Phenol-, Dimethylphenyl-, Dimethoxyphenyl-, Methylendioxyphenylrest,
- einen Amid- oder Aminrest enthält, bedeutet, zur Verwendung als Medikament verwendet.

**4.** Verfahren zur Herstellung eines substituierten Chinoleins, dadurch gekennzeichnet, daß man ein Chinolein der Formel (I) nach Anspruch 1 herstellt, worin

- $R_3$ , $R_4$ , $R_5$ , $R_6$ und $R_7$ Wasserstoffatome sind,
- $R_1$ eine Methoxygruppe ist,
- $R_2$ eine Propylgruppe ist.

**5.** Verfahren zur Herstellung eines substituierten Chinoleins, dadurch gekennzeichnet, daß man ein Chinolein der Formel (I), wie in Anspruch 1 definiert, herstellt, worin

- $R_1$ , $R_3$ , $R_4$ , $R_5$ , $R_6$ und $R_7$ Wasserstoffatome sind,
- $R_2$ eine Trans-Epoxypropylgruppe ist.

**6.** Verfahren zur Herstellung eines Medikaments, dadurch gekennzeichnet, daß man als Wirkstoff des Medikaments ein substituiertes Chinolein der Formel (I) nach Anspruch 1, worin

- $R_1$ , $R_3$ , $R_4$ , $R_5$ , $R_6$ und $R_7$ Wasserstoffatome sind,
- $R_2$ eine Propylgruppe ist

  verwendet.

**7.** Verfahren zur Herstellung eines Medikaments, dadurch gekennzeichnet, daß man als Wirkstoff des Medikaments ein substituiertes Chinolein der Formel (I), wie in Anspruch 1 definiert, worin

- $R_1$ , $R_3$ , $R_4$ , $R_5$ , $R_6$ und $R_7$ Wasserstoffatome sind,

- $R_2$ eine Propenylgruppe ist

verwendet.

8. Verfahren nach Anspruch 1, dadurch gekennzeichnet, daß man als Wirkstoff des Medikaments mindestens ein Chinolein, wie in einem der Ansprüche 2 bis 7 definiert, verwendet.

9. Verfahren nach Anspruch 1, dadurch gekennzeichnet, daß das Medikament gegen die Leishmaniose für die orale Verabreichung bestimmt ist.

## Claims

**Claims for the following Contracting States : AT, BE, CH, LI, DE, DK, FR, GB, IE, IT, LU, MC, NL, SE**

1. Use of at least one substituted quinoline of general formula (I)

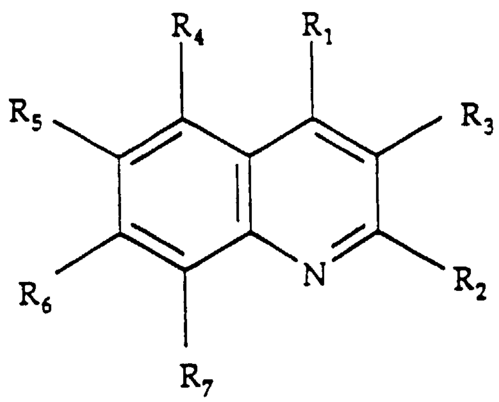

(I)

in which $R_1$, $R_3$, $R_4$, $R_5$, $R_6$ and $R_7$ each represent, independently, a hydrogen atom, a linear or branched $C_1$ to $C_7$ alkyl, alkenyl, epoxyalkyl or mono- or polyhydric alcohol group, an amine group or an amide group, a group OR in which R represents hydrogen or a $C_{1-7}$ alkyl or alkenyl group or a phenyl group; and $R_2$ represents:

- a group OR in which R has the same meaning as above, or alternatively
- a $C_1$ to $C_7$ alkyl, alkenyl or epoxyalkyl group,
- a phenyl group, a phenol, methylenedioxyphenyl or dimethoxyphenyl group, or alternatively
- a $C_{1-7}$ alkyl, alkenyl or epoxyalkyl group bearing at least one of the following substituents: a $C_{1-4}$ alkyl or alkenyl group; a phenyl, phenol, dimethylphenyl, dimethoxyphenyl or methylenedioxyphenyl group; or a group OR' in which R' represents hydrogen or a $C_{1-4}$ alkyl or alkenyl group; or a group NHR" in which R" represents hydrogen or a $C_{1-4}$ alkyl or alkenyl group; or an amide group, or alternatively
- $R_2$ and $R_3$ together form a furan ring; as well as a salt or a derivative of said quinoline for obtaining an antileishmanial medicinal product.

2. A substituted quinoline of formula (I) as defined in claim 1, wherein $R_3$, $R_4$ and $R_5$ represent a hydrogen atom and $R_1$, $R_6$ and $R_7$ represent a hydrogen atom or a methoxy group, for use as a medicinal product.

3. A substituted quinoline of formula (I) as defined in claim 1, wherein $R_2$ is an ethyl, ethylene or epoxyethyl group bearing at least one of the following substituents:

- a $C_{1-4}$ alkyl or alkenyl group, or
- a group OR' in which R' represents hydrogen or a $C_{1-4}$ alkyl or alkenyl group,

- a phenyl, phenol, dimethylphenyl, dimethoxyphenyl or methylenedioxyphenyl group,
- an amide or amine group,

for use as a medicinal product.

**4.** A substituted quinoline of formula (I) as defined in claim 1, in which:

- $R_3$, $R_4$, $R_5$, $R_6$ and $R_7$ are hydrogen atoms;
- $R_1$ is a methoxy group;
- $R_2$ is a propyl group.

**5.** A substituted quinoline of formula (I) as defined in claim 1, in which:

- $R_1$, $R_3$, $R_4$, $R_5$, $R_6$ and $R_7$ are hydrogen atoms;
- $R_2$ is a trans-epoxypropyl group.

**6.** A substituted quinoline of formula (I) as defined in claim 1, in which:

- $R_1$, $R_3$, $R_4$, $R_5$, $R_6$ and $R_7$ are hydrogen atoms;
- $R_2$ is a propyl group,

for use as a medicinal product.

**7.** A substituted quinoline of formula (I) as defined in claim 1, in which:

- $R_1$, $R_3$, $R_4$, $R_5$, $R_6$ and $R_7$ are hydrogen atoms;
- $R_2$ is a propenyl group,

for use as a medicinal product.

**8.** A pharmaceutical composition for the treatment of leishmaniasis, which comprises as active principle at least one quinoline as defined in any one of claims 2 to 7.

**9.** The pharmaceutical composition for the treatment of leishmaniasis as claimed in claim 8, which is intended for oral administration.

**10.** Use according to claim 1, wherein said antileishmanial medicinal product is intended for oral administration.

**Claims for the following Contracting States : ES, GR**

**1.** A process for the preparation of an antileishmanial medicinal product, wherein a substituted quinoline of general formula (I)

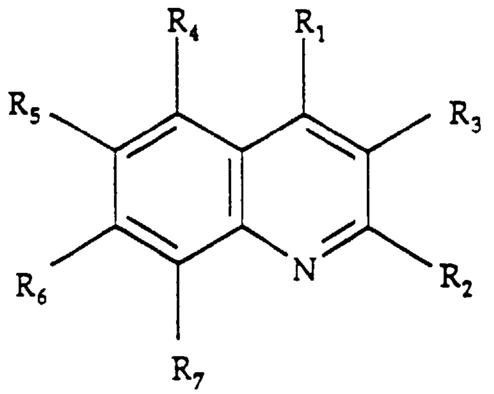

(I)

in which $R_1$, $R_3$, $R_4$, $R_5$, $R_6$ and $R_7$ each represent, independently, a hydrogen atom, a linear or branched $C_1$ to $C_7$ alkyl, alkenyl, epoxyalkyl or mono- or polyhydric alcohol group, an amine group or an amide group, a group OR in which R represents hydrogen or a $C_{1-7}$ alkyl or alkenyl group or a phenyl group; and $R_2$ represents:

- a group OR in which R has the same meaning as above, or alternatively
- a $C_1$ to $C_7$ alkyl, alkenyl or epoxyalkyl group,
- a phenyl group, a phenol, methylenedioxyphenyl or dimethoxyphenyl group, or alternatively
- a $C_{1-7}$ alkyl, alkenyl or epoxyalkyl group bearing at least one of the following substituents: a $C_{1-4}$ alkyl or alkenyl group; a phenyl, phenol, dimethylphenyl, dimethoxyphenyl or methylenedioxyphenyl group; or a group OR' in which R' represents hydrogen or a $C_{1-4}$ alkyl or alkenyl group; or a group NHR" in which R" represents hydrogen or a $C_{1-4}$ alkyl or alkenyl group; or an amide group, or alternatively.
- $R_2$ and $R_3$ together form a furan ring; as well as a salt or a derivative of said quinoline,

is used as active constituent of said medicinal product.

**2.** A process for the preparation of a medicinal product, wherein a substituted quinoline of formula (I) as defined in claim 1, in which $R_3$, $R_4$ and $R_5$ represent a hydrogen atom and $R_1$, $R_6$ and $R_7$ represent a hydrogen atom or a methoxy group, is used as active constituent of said medicinal product.

**3.** A process for the preparation of a medicinal product, wherein a substituted quinoline of formula (I) as defined in claim 1, in which $R_2$ is an ethyl, ethylene or epoxyethyl group bearing at least one of the following substituents:

- a $C_{1-4}$ alkyl or alkenyl group, or
- a group OR' in which R' represents hydrogen or a $C_{1-4}$ alkyl or alkenyl group,
- a phenyl, phenol, dimethylphenyl, dimethoxyphenyl or methylenedioxyphenyl group,
- an amide or amine group,

is used as active constituent of said medicinal product.

**4.** A process for the preparation of a substituted quinoline, wherein a quinoline of formula (I) as defined in claim 1, in which:

- $R_3$, $R_4$, $R_5$, $R_6$ and $R_7$ are hydrogen atoms;
- $R_1$ is a methoxy group;
- $R_2$ is a propyl group,

is prepared.

**5.** A process for the preparation of a substituted quinoline, wherein a quinoline of formula (I) as defined in claim 1, in which:

- $R_1$, $R_3$, $R_4$, $R_5$, $R_6$ and $R_7$ are hydrogen atoms;

- $R_2$ is a trans-epoxypropyl group,

is prepared.

**6.** A process for the preparation of a medicinal product, wherein a substituted quinoline of formula (I) as defined in claim 1, in which:

- $R_1$, $R_3$, $R_4$, $R_5$, $R_6$ and $R_7$ are hydrogen atoms;
- $R_2$ is a propyl group,

is used as active constituent of said medicinal product.

**7.** A process for the preparation of a medicinal product, wherein a substituted quinoline of formula (I) as defined in claim 1, in which:

- $R_1$, $R_3$, $R_4$, $R_5$, $R_6$ and $R_7$ are hydrogen atoms;
- $R_2$ is a propenyl group,

is used as active constituent of said medicinal product.

**8.** A process according to claim 1, wherein at least one quinoline as defined in any one of claims 2 to 7 is used as active constituent of said medicinal product.

**9.** A process according to claim 1, wherein said antileishmanial medicinal product is intended for oral administration.

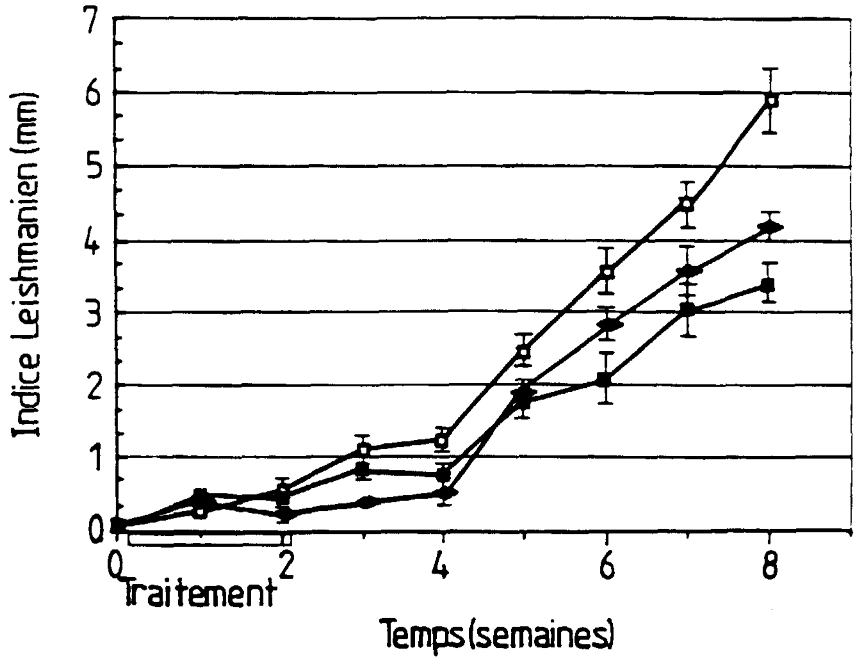
Indice Leishmanien (mm)
7
6
5
4
3
2
1
0
0
2
4
6
8
Traitement
Temps(semaines)

FIG.1a

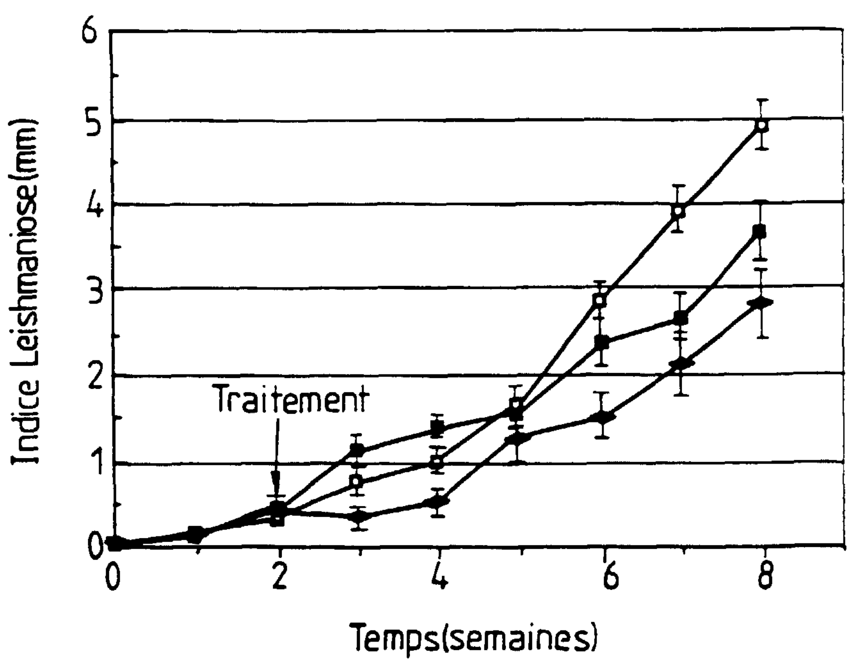
Indice Leishmaniose(mm)
6
5
4
3
2
1
0
Traitement
0
2
4
6
8
Temps(semaines)

FIG.1b

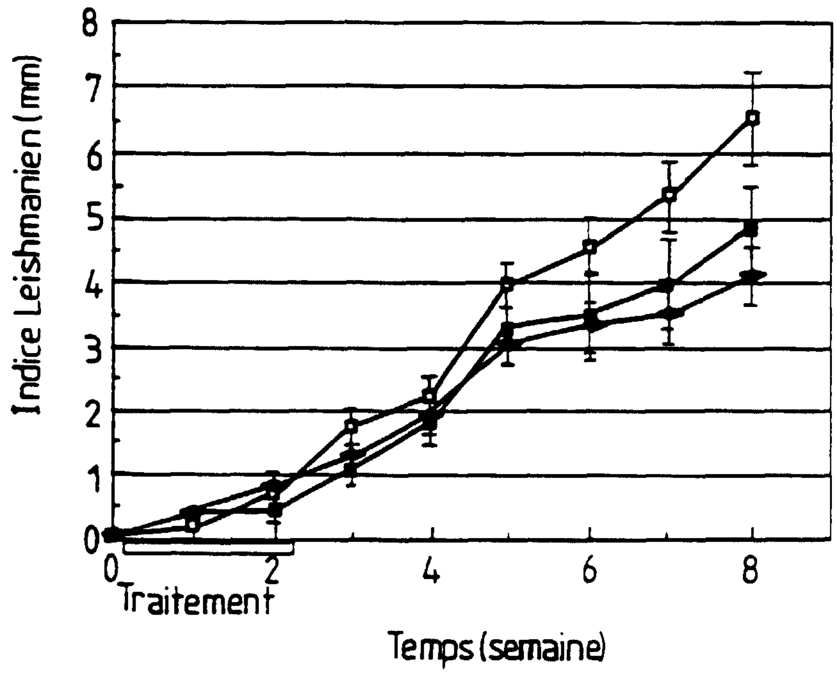
Indice Leishmanien (mm)
8
7
6
5
4
3
2
1
0
0
2
4
6
8
Traitement
Temps (semaine)

FIG. 2

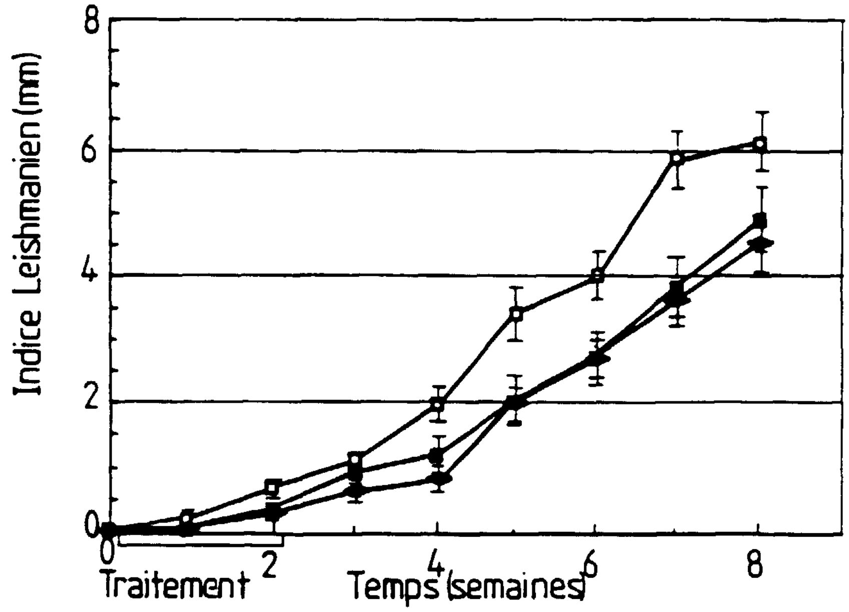

8
6
4
2
0
Indice Leishmanien (mm)
0
2
4
6
8
Traitement
Temps (semaines)


FIG. 3a

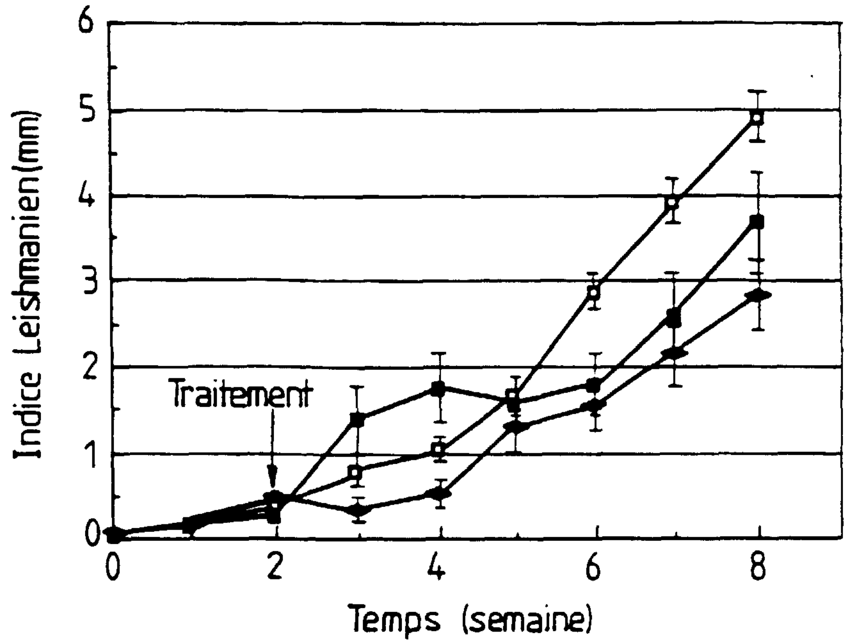

6
5
4
3
2
1
0
Indice Leishmanien (mm)
Traitement
0
2
4
6
8
Temps (semaine)


FIG. 3b

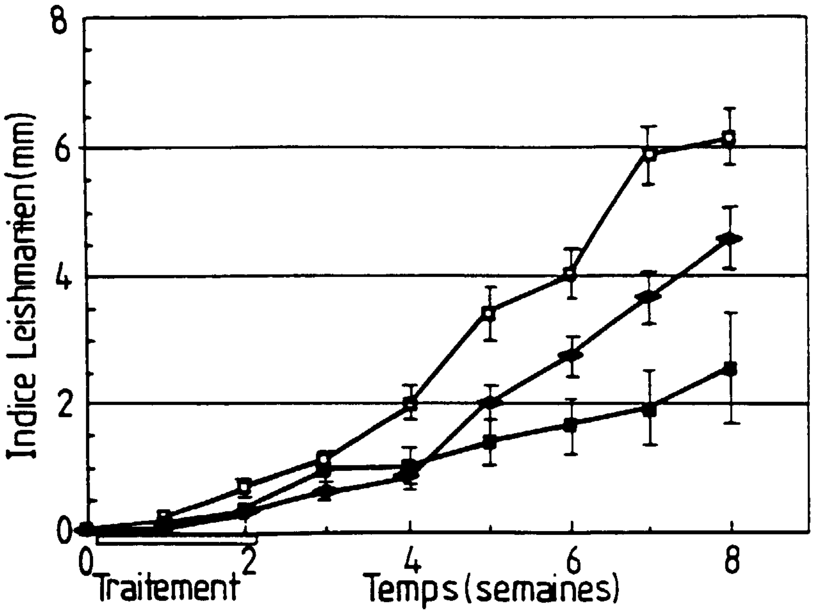

Indice Leishmanien (mm)
8
6
4
2
0
0
2
4
6
8
Traitement
Temps (semaines)


FIG. 4